(19) Europäisches Patentamt
European Patent Office
Office européen des brevets

(11) Veröffentlichungsnummer: **0 045 520**
**B1**

(12) **EUROPÄISCHE PATENTSCHRIFT**

(45) Veröffentlichungstag der Patentschrift: **20.06.84**

(21) Anmeldenummer: **81106138.1**

(22) Anmeldetag: **05.08.81**

(51) Int. Cl.³: **C 07 D 487/04,** A 61 K 31/55 // C07C49/233, C07C97/10, C07D209/48, C07D233/16, (C07D487/04, 239/00, 223/00)

(54) **Pyrimido(4,5-d)(2)benzazepine, Verfahren für ihre Herstellung und diese enthaltende Arzneimittel.**

(30) Priorität: **05.08.80 US 175554**

(43) Veröffentlichungstag der Anmeldung: **10.02.82 Patentblatt 82/6**

(45) Bekanntmachung des Hinweises auf die Patenterteilung: **20.06.84 Patentblatt 84/25**

(84) Benannte Vertragsstaaten: **BE CH DE FR GB IT LI NL SE**

(56) Entgegenhaltungen:
**EP - A - 0 014 470**
**DE - A - 2 524 048**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Fryer, Rodney Ian, 5 Eton Drive, North Caldwell, N.J. (US)**
Erfinder: **Trybulski, Eugene J., 13 Homer Street, Parsippany, N.J. (US)**
Erfinder: **Walser, Armin, 19 Crane Avenue, West Caldwell, N.J. (US)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft Benzazepinderivate. Im speziellen betrifft sie Pyrimido-[4,5-d]-[2]-benzazepine der allgemeinen Formel

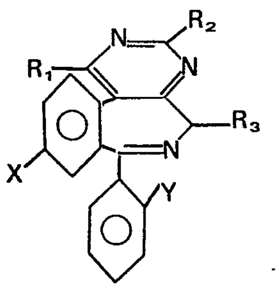

(I)

worin $R_1$ Wasserstoff, niederes Alkyl, Chlor, Brom, niederes Alkoxy, Hydroxy, niederes Alkylthio oder $-NR_4R_5$, $R_4$ und $R_5$ Wasserstoff, niederes Alkyl oder diniederes alkylaminoniederes Alkyl, $R_2$ Wasserstoff, niederes Alkyl oder Amino, $R_3$ Wasserstoff, Acyloxy oder Hydroxy, X Halogen mit einer Ordnungszahl, welche nicht grösser als 35 ist, und Y Wasserstoff oder Halogen mit einer Ordnungszahl, welche nicht grösser als 35 ist, bedeuten, und die 6-Oxide davon, wenn $R_3$ Wasserstoff bedeutet, und pharmazeutisch annehmbare Salze davon.

In DE-Al Nr. 2524048 sind 6-Aryl-1,4-dihydro- oder -2,4-dihydropyrazolo-[4,3-d]-[2]-benzazepine beschrieben, welche antidepressive Effekte vom Imipramintypus und insbesondere anxiolytische Wirkungen von langer Dauer zeigen.

Die in der vorliegenden Beschreibung verwendeten Ausdrücke Halo oder Halogen beziehen sich auf Chlor, Brom oder Fluor.

Der Ausdruck niederes Alkyl bezeichnet geradkettige oder verzweigte $C_1$- bis $C_7$-Kohlenwasserstoffreste, wie Methyl, Äthyl, Propyl, Isopropyl, Butyl etc.

Der Ausdruck Acyloxy bezeichnet einen von einer organischen Säure durch Entfernen eines Wasserstoffatoms abgeleiteten Rest, wie Acetyloxy, Benzoyloxy etc.

Die Verbindungen der Formel I, die N-Oxide von Verbindungen der Formel I, worin $R_3$ Wasserstoff bedeutet, und ihre pharmazeutisch annehmbaren Säureadditionssalze können erfindungsgemäss hergestellt werden, indem man

a) eine Verbindung der allgemeinen Formel

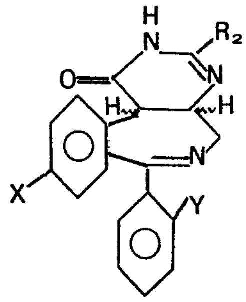

(VI)

worin $R_2$, X und Y obige Bedeutung besitzen, dehydriert, oder

b) eine Verbindung der Formel I, worin $R_3$ Wasserstoff und $R_1$ Wasserstoff, niederes Alkyl, Chlor, Brom, niederes Alkoxy, Hydroxy oder $-NR_4R_5$ bedeuten, und $R_4$ und $R_5$ obige Bedeutung besitzen, in der 6-Stellung oxydiert, oder

c) ein 6-Oxid einer Verbindung der Formel I, worin $R_3$ Wasserstoff bedeutet, mit einem Acylierungsmittel behandelt, oder

d) eine Verbindung der Formel I, worin $R_3$ Acyloxy bedeutet, einer alkalischen Hydrolyse unterwirft, oder

e) eine Verbindung der Formel I, worin $R_1$ Hydroxy und $R_3$ Wasserstoff bedeuten, chloriert oder bromiert, oder

f) eine Verbindung der Formel I, worin $R_1$ Chlor oder Brom bedeutet, oder ein 6-Oxid davon, wenn $R_3$ Wasserstoff bedeutet, mit einem einen niederen Alkyl-, niederen Alkylmercapto- oder niederen Alkoxyrest, oder einem die Gruppe $-NR_4R_5$ liefernden Mittel behandelt, oder

g) eine Verbindung der Formel I, worin $R_1$ Chlor oder Brom bedeutet, oder ein 6-Oxid davon, wenn $R_3$ Wasserstoff bedeutet, reduziert, oder

h) eine Verbindung der Formel I, worin $R_1$ niederes Alkylmercapto und $R_3$ Wasserstoff bedeuten, entschwefelt, oder

i) eine Verbindung der Formel I, worin $R_1$ niederes Alkylmercapto und $R_3$ Wasserstoff bedeuten, mit einem Amin der Formel $HNR_4R_5$, worin $R_4$ und $R_5$ obige Bedeutung besitzen, behandelt, oder

j) eine Verbindung der Formel I oder ein 6-Oxid einer Verbindung der Formel I, worin $R_3$ Wasserstoff bedeutet, in ein pharmazeutisch annehmbares Säureadditionssalz überführt.

Die verschiedenen Verfahrensvarianten und die Herstellung der darin benötigten Ausgangsstoffe werden durch die nachfolgenden Reaktionsschemata illustriert

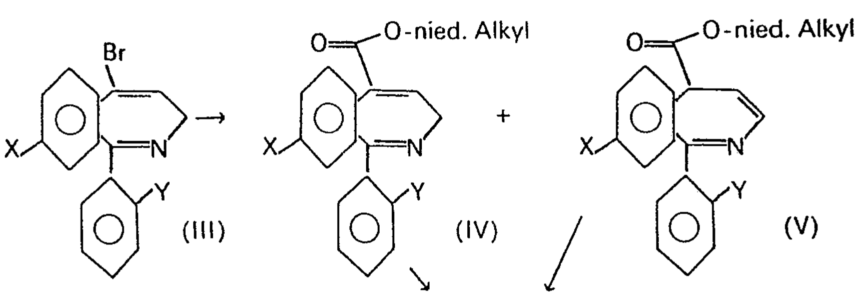

(III) (IV) + (V)

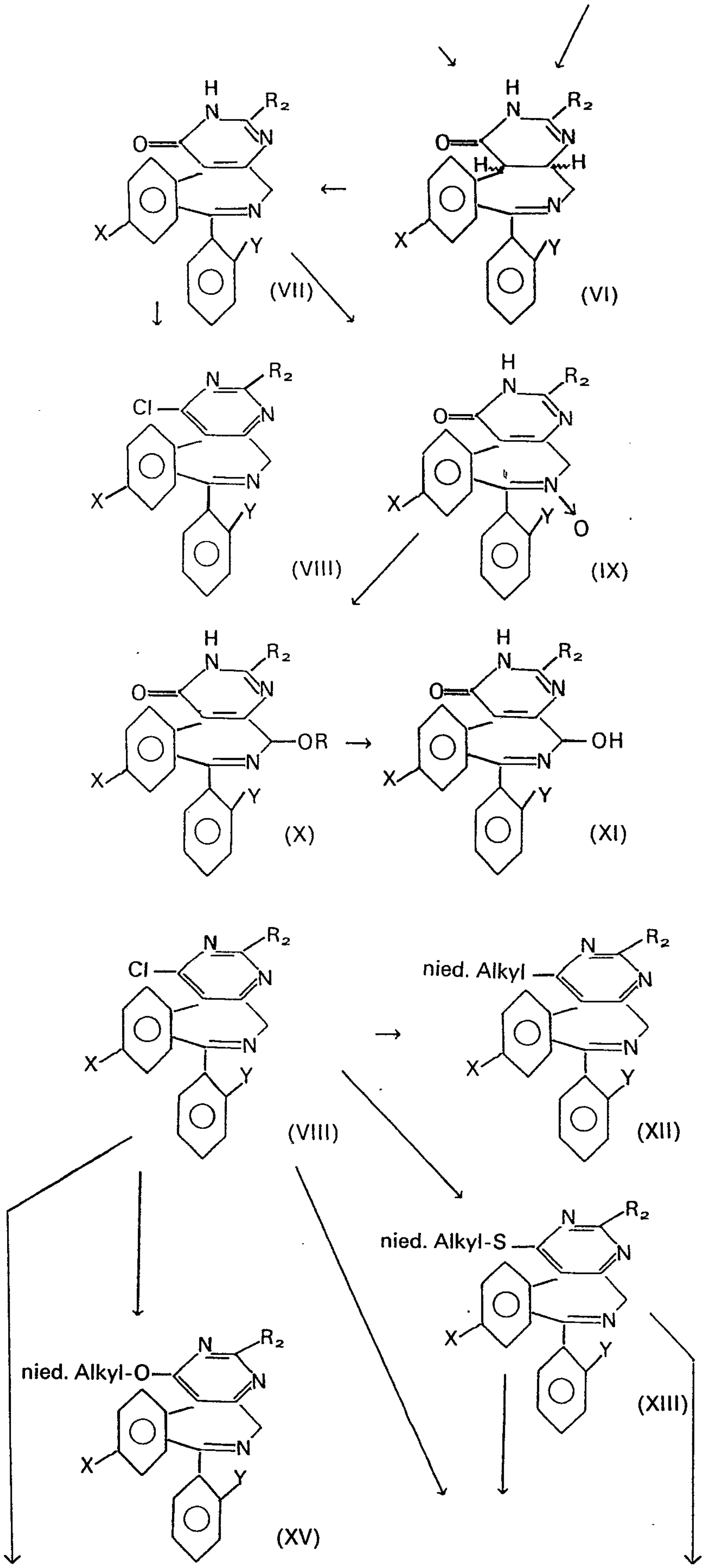

(VII) (VI) (VIII) (IX) (X) (XI) (VIII) (XII) (XIII) (XV)

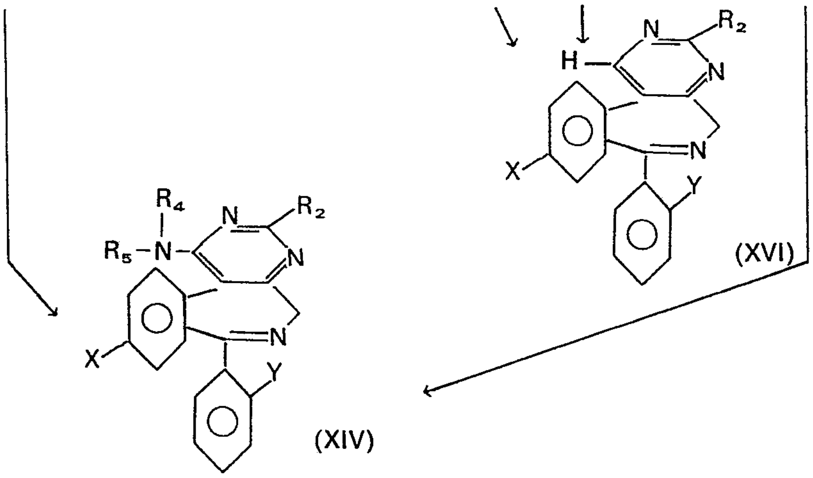

(XVI)

(XIV)

worin $R_2$, $R_4$, $R_5$, X und Y obige Bedeutung besitzen, und R Acyl bedeutet.

Eine Verbindung der Formel III kann aus einem Jodbenzophenon hergestellt werden, das die geeigneten Substituenten X und Y besitzt. Die Jodbenzophenonderivate können hergestellt werden, indem man ein entsprechendes, bekanntes Aminobenzophenon unter Verwendung von Natriumnitrit in Schwefelsäure diazotiert, die erhaltenen Salze durch Ausfällen der entsprechenden Tetrafluoroborate isoliert, diese anschliessend in Wasser aufschlämmt und mit wässerigem Kaliumjodid behandelt. Diese Reaktionen werden nach an sich bekannten Methoden durchgeführt.

Die Jodbenzophenonderivate können anschliessend in Gegenwart von Palladiumchlorid oder -acetat, Kupfer(I)jodid, einem Organophosphin, z.B. Triphenylphosphin, und einem tertiären oder sekundären Amin, wie Diäthylamin oder Diisopropylamin, mit Propargylphthalimid umgesetzt werden. Geeignete Lösungsmittel sind die obigen Amine, z.B. Diäthylamin, halogenierte Kohlenwasserstoffe, z.B. Methylenchlorid, Dimethylformamid oder ätherische Lösungsmittel. Die Reaktionstemperatur variiert in einem Bereich von 0 bis 70°C und liegt vorzugsweise bei etwa Raumtemperatur.

Die Gegenwart von Kupfer(I)jodid ist zwingend, wenn die Reaktion bei Raumtemperatur oder unterhalb davon durchgeführt wird, nicht jedoch wenn die Reaktion unter Erwärmen durchgeführt wird. Die Gegenwart eines Organophosphins ist nicht absolut notwendig, jedoch sehr vorteilhaft. Anstelle eines Palladiumsalzes und eines Organophosphins kann man auch einen geeigneten Komplex, wie Dichlorbis(triphenylphosphin)palladium(II), verwenden.

Das erhaltene Produkt kann unter Verwendung eines Lindlarkatalysators (10% Palladium auf Bariumsulfat, vorhydriert) bei etwa Raumtemperatur und Atmosphärendruck hydriert werden. Unter den geeigneten Lösungsmitteln sind $C_1$- bis $C_6$-Alkohole, Tetrahydrofuran, Dioxan oder Toluol.

Der erhaltene Stoff kann anschliessend mit einer wässerigen Lösung von einem niederen Alkylamin, z.B. Methylamin, umgesetzt werden. Als Lösungsmittel verwendet man dabei einen $C_1$- bis $C_4$-Alkohol, vorzugsweise Äthanol. Die Reaktion wird vorteilhafterweise bei etwa Raumtemperatur durchgeführt. Das in einem ersten Schritt gebildete offenkettige Amin wird nicht isoliert, es cyclisiert spontan.

Es ist auch möglich, die obige Reaktion mit einer wässerigen Lösung von einem niederen Alkylamin vor der Hydrierung unter Verwendung eines Lindlarkatalysators durchzuführen.

Das erhaltene Produkt kann anschliessend mit einem Halogenierungsmittel, wie elementares Chlor, Brom oder Jod, in einem halogenierten Kohlenwasserstoff, wie Methylenchlorid oder Chloroform, halogeniert werden. Die Reaktion wird in einem Bereich von 0°C bis Raumtemperatur durchgeführt; man arbeitet vorzugsweise bei etwa Raumtemperatur.

Das erhaltene Produkt kann anschliessend zu einer Verbindung der Formel III dehydrohalogeniert werden, und zwar unter Verwendung eines Alkalimetall-, z.B. Kalium- oder Natrium-, -hydroxydes, -carbonates oder -alkoxides. Unter den geeigneten Lösungsmitteln sind $C_1$- bis $C_6$-Alkohole, Tetrahydrofuran, Dioxan und Dimethylformamid. Die Reaktionstemperatur variiert in einem Bereich von 0°C bis Siedetemperatur des gewählten Lösungsmittels und liegt vorzugsweise bei etwa Raumtemperatur.

*III → IV + V*

Eine Verbindung der Formel III kann in Gegenwart eines $C_1$- bis $C_6$-Alkoholes, eines tertiären Amins, z.B. von Triäthylamin oder Tri-n-butylamin, einem Palladiumkatalysator, wie Palladiumacetat oder Palladiumbromid und Triphenylphosphin oder Dibrombis(triphenylphosphin)palladium(II), und gegebenenfalls Kupfer(I)jodid oder Hydrazin mit Kohlenmonoxid umgesetzt werden. Die Reaktionstemperatur variiert in einem Bereich von 60 bis 120°C und liegt vorzugsweise bei etwa 100°C. Die Reaktion kann gegebenenfalls unter Druck durchgeführt werden, um die Reaktionszeit abzukürzen.

*IV + V → VI*

Die Verbindungen der Formeln IV und V können mit einem Amidin, wie Formamidin, Acetamidin

oder Guanidin, in Gegenwart einer Base, wie einem Alkalimetallalkoxid, z.B. Natrium- oder Kaliummethoxid, -äthoxid oder -butoxid, umgesetzt werden. Unter den geeigneten Lösungsmitteln sind $C_1$- bis $C_4$-Alkohole. Die Reaktionstemperatur kann in einem Bereich von Raumtemperatur bis Siedetemperatur variieren, wobei die bevorzugte Temperatur vom gewählten Amidin abhängt, jedoch nicht mehr als 100°C beträgt.

*VI → VII*

Eine Verbindung der Formel VI kann mit einem Oxidationsmittel, wie Mangandioxid, in einem geeigneten Lösungsmittel, z.B. in Methylenchlorid, Tetrahydrofuran oder in Dimethylformamid, umgesetzt werden. Die Reaktionstemperatur kann in einem Bereich von Raumtemperatur bis 100°C variieren und liegt vorzugsweise bei etwa 60°C.

Eine Oxoverbindung der Formel VII ist eine tautomere Form der entsprechenden Verbindung der Formel I, worin $R_1$ Hydroxy bedeutet. Dasselbe gilt für die Verbindungen der Formeln X und XI. Eine Oxoverbindung der Formel IX ist eine tautomere Form eines 6-Oxides einer entsprechenden Verbindung der Formel I, worin $R_1$ Hydroxy bedeutet.

*VII → IX*

Eine Verbindung der Formel VII kann mit einer Persäure, wie m-Chlorperbenzoesäure oder Pertrifluoressigsäure, in einem halogenierten Kohlenwasserstoff, wie Methylenchlorid, umgesetzt werden. Die Reaktionstemperatur kann in einem Bereich von −20°C bis Raumtemperatur variieren und liegt vorzugsweise bei etwa 0°C.

*IX → X*

Eine Verbindung der Formel VIII kann mit einem von einer $C_1$- bis $C_6$-Carbonsäure abgeleiteten Säureanhydrid, wie Essigsäureanhydrid, Propionsäureanhydrid etc. oder Trifluoressigsäureanhydrid, umgesetzt werden. Die Reaktionstemperatur kann in einem Bereich von 40°C bis Siedetemperatur variieren und liegt vorzugsweise bei etwa Siedetemperatur. Die Reaktionstemperatur sollte 125°C nicht übersteigen.

*X → XI*

Eine Verbindung der Formel X kann mit einem wässerigen Alkalimetallcarbonat, wie Natrium- oder Kaliumcarbonat, oder einem wässerigen Alkalimetallhydroxid, wie Natrium- oder Kaliumhydroxid, umgesetzt werden. Gegebenenfalls kann man einen Lösungsvermittler, wie einen $C_1$- bis $C_6$-Alkohol oder Tetrahydrofuran, verwenden. Die Reaktionstemperatur kann in einem Bereich von 0 bis 100°C variieren und liegt vorzugsweise zwischen Raumtemperatur und 100° C.

*VII → VIII*

Eine Verbindung der Formel VII kann mit einem Phosphorylhalogenid, z.B. einem Chlorid oder Bromid, umgesetzt werden. Man kann gegebenenfalls einen Lösungsvermittler, wie einen chlorierten Kohlenwasserstoff, z.B. Methylenchlorid oder Chloroform, verwenden. Die Reaktionstemperatur kann in einem Bereich von Raumtemperatur bis Siedetemperatur des gewählten Lösungsmittels variieren. Vorzugsweise arbeitet man bei etwa Raumtemperatur.

*VIII → XII*

Eine Verbindung der Formel VIII oder eine entsprechende 1-Bromverbindung kann mit einem Organometallreagens, wie einem niederen Alkyllithium oder einem niederen Alkylcuprat, in einem ätherischen Lösungsmittel, wie Diäthyläther oder Tetrahydrofuran, umgesetzt werden. Die Reaktionstemperatur kann in einem Bereich von −78 bis 0°C variieren und liegt vorzugsweise bei etwa −78°C.

*VIII → XIII*

Eine Verbindung der Formel VIII oder eine entsprechende 1-Bromverbindung kann mit einem Alkalimetallsalz eines niederen Alkylmercaptans in einem polaren Lösungsmittel, wie Dimethylformamid oder Dimethylsulfoxid, umgesetzt werden. Die Reaktionstemperatur kann in einem Bereich von −78°C bis Raumtemperatur variieren und liegt vorzugsweise bei etwa Raumtemperatur.

*VIII → XIV und XIII → XIV*

Eine Verbindung der Formel VIII oder eine entsprechende 1-Bromverbindung kann mit Ammoniak oder einem primären oder sekundären Amin in einem polaren Lösungsmittel, wie Dimethylformamid oder Dimethylsulfoxid umgesetzt werden. Die Reaktionstemperatur kann in einem Bereich von Raumtemperatur bis 100°C variieren und liegt vorzugsweise bei etwa 60°C. Auf ähnliche Weise kann eine Verbindung der Formel XIII zu einer Verbindung der Formel XIV umgesetzt werden.

*VIII → XV*

Eine Verbindung der Formel VIII oder eine entsprechende 1-Bromverbindung kann mit einem Alkalimetallalkoxid im entsprechenden $C_1$- bis $C_6$-Alkohol umgesetzt werden. Gegebenenfalls kann man einen Lösungsvermittler, wie einen Äther, z.B. Tetrahydrofuran, Dioxan etc., oder Dimethylformamid verwenden. Die Reaktionstemperatur kann in einem Bereich von 0°C bis Raumtemperatur variieren und liegt vorzugsweise bei etwa 0°C.

Man kann eine Verbindung der Formel VIII oder eine entsprechende 1-Bromverbindung in Analogie zur Stufe VII → IX zum entsprechenden 6-Oxid der Chlor- oder Bromverbindung umsetzen, und dieses anschliessend in Analogie zu den Stufen IX → X und X → XI zu Analogen von Verbindungen der Formeln X und XI umsetzen. Die Chlor- und Bromsubstituenten in diesen Analogen können anschliessend wie in Formelschema I beschrieben durch verschiedene Reste $R_1$ ersetzt werden, und zwar in Analogie zu den Stufen VIII → XII, VIII → XIII, VIII → XVI, VIII → XV und VIII → XIV.

Darüber hinaus können Verbindungen der Formeln XII, XIV, XV und XVI in Analogie zur Stufe VII → IX umgesetzt werden; und die 6-Oxide von Verbindungen der Formeln XII, XIII, XIV, XV und XVI können in Analogie zur Stufe IX → X und X → XI zu Analogen von Verbindungen der Formeln X und XI umgesetzt werden.

*VIII → XVI*

Eine Verbindung der Formel VIII kann mit einem Reduktionsmittel, wie Wasserstoff, bei Atmosphärendruck unter Verwendung von Raney-Nickel als Katalysator und in einem $C_1$- bis $C_4$-Alkohol, oder Zink und Ammoniumchlorid in einer Mischung aus Wasser und Tetrahydrofuran oder Wasser und Dioxan umgesetzt werden. Die Reaktionstemperatur variiert in einem Bereich von 0°C bis Siedetemperatur des gewählten Lösungsmittels und liegt vorzugsweise bei Raumtemperatur.

*XIII → XVI*

Eine Verbindung der Formel XIII kann in einem $C_1$- bis $C_4$-Alkohol mit Raney-Nickel umgesetzt werden. Die Reaktionstemperatur variiert in einem Bereich von 0°C bis Siedetemperatur des gewählten Lösungsmittels und liegt vorzugsweise bei etwa Siedetemperatur. Bei dieser Reaktion handelt es sich um eine dem Fachmann geläufige Entschwefelung.

Der Ausdruck pharmazeutisch annehmbare Salze umfasst sowohl Salze mit anorganischen als auch mit organischen, pharmazeutisch annehmbaren starken Säuren, wie Schwefel-, Salz-, Salpeter-, Methansulfon-, und p-Toluolsulfonsäure. Solche Salze können vom Fachmann sehr leicht hergestellt werden, wenn er sich den Stand der Technik und die Natur der in Salze zu überführenden Verbindung vergegenwärtigt.

Die erfindungsgemässen Pyrimido-[4,5-d]-[2]-benzazepine sind nützlich als Heilmittel und zeichnen sich durch sedative und anxiolytische Eigenschaften aus. Diese Verbindungen können in Form üblicher pharmazeutischer Zubereitungen verwendet werden; beispielsweise können sie mit üblichen organischen oder anorganischen, inerten, für parenterale oder enterale Verabreichung geeigneten Trägerstoffen vermischt werden, beispielsweise mit Wasser, Gelatine, Milchzucker, Stärke, Magnesiumstearat, Talk, pflanzlichen Ölen, Gummiarabikum, Polyalkylenglykolen, Vaselin od. dgl. Sie können als übliche pharmazeutische Formen verabreicht werden, beispielsweise als feste Formen, wie Tabletten, Dragées, Kapseln, Suppositorien od. dgl., oder als flüssige Formen, wie Lösungen, Suspensionen oder Emulsionen. Darüber hinaus können die erfindungsgemässe Verbindungen erhaltenden pharmazeutischen Zubereitungen üblichen pharmazeutischen Operationen, wie Sterilisiereung, unterworfen werden, und sie können übliche pharmazeutische Hilfsstoffe enthalten, wie Konservierungsmittel, Stabilisierungsmittel, Netzmittel, Emulgiermittel, Salze zur Veränderung des osmotischen Druckes oder Puffer. Die pharmazeutischen Präparate können auch noch andere therapeutisch wertvolle Stoffe enthalten.

Eine geeignete pharmazeutische Dosierungseinheit kann zwischen 1 bis 500 mg einer Verbindung der Formel I enthalten, wobei ein Dosierungsbereich von 1 bis 100 mg für orale Verabreichung und ein Dosierungsbereich von 1 bis 50 mg für parenterale Verabreichung bevorzugt wird. In jedem Einzelfall muss jedoch die Person, welche die Verabreichung der fraglichen Verbindungen durchführt oder überwacht, die Dosierung nach ihrem fachlichen Urteil den individuellen Erfordernissen anpassen. Es ist zu beachten, dass die vorstehenden Dosierungen lediglich im Sinne von Beispielen angegeben sind und dass sie den Umfang und die Ausführung der vorliegenden Erfindung in keiner Weise einschränken.

Der Ausdruck Dosierungseinheit, wie er in der vorliegenden Beschreibung verwendet wird, bezeichnet einzelne pharmazeutische Einheiten, welche sich als Einheitsdosen für Säuger eignen und von welchen jede eine vorbestimmte Menge des Wirkstoffes enthält, welche so berechnet wurde, dass sie zusammen mit dem erforderlichen pharmazeutischen Verdünnungsmittel, Träger oder Vehikel den erwünschten therapeutischen Effekt zur Folge hat.

Bevorzugte Verbindungen der Formel I sind solche, worin $R_1$ obige Bedeutung besitzt, $R_3$ Wasserstoff und $R_2$ Wasserstoff oder niederes Alkyl bedeuten.

Ganz besonders bevorzugte Verbindungen der Formel I sind

A) 9-Chlor-3-methyl-7-phenyl-5H-pyrimido-[4,5-d]-[2]-benzazepin-1(2H)-on,

B) 9-Chlor-7-(2-fluorphenyl)-3-methyl-5H-pyrimido-[4,5-d]-[2]-benzazepin-1(2H)-on,

C) 1,9-Dichlor-3-methyl-7-phenyl-5H-pyrimido-[4,5-d]-[2]-benzazepin,

D) 9-Chlor-1,3-dimethyl-7-phenyl-5H-pyrimido-[4,5-d]-[2]-benzazepin, und

E) 9-Chlor-7-(2-fluorphenyl)-4-methyl-1-methoxy-5H-pyrimido-[4,5-d]-[2]-benzazepin.

Die pharmakologischen Eigenschaften der erfindungsgemässen Verbindungen sollen durch die nachfolgenden, für eine erfindungsgemässe Verbindung ermittelten Daten illustriert werden.

*Versuche*

| Verbindung | Kampftest | Test an der geneigten Ebene | Test an der wachen Katze |
|---|---|---|---|
| 1,9-Dichlor-3-methyl-7-phenyl-5H-pyrimido-[4,5-d]-[2]-benzazepin Toxizität ($DL_{50}$) = | 10 mg/kg >1000 mg/kg *(p.o.)* | 400 mg/kg | 10 mg/kg |

Es folgt eine kurze Beschreibung der Testversuche.

*Kampftest:*

Man bringt zwei Mäuse unter ein umgekehrtes 1-l-Becherglas auf ein Gitter, durch welches ihnen Schläge auf die Füsse versetzt werden. Dadurch geraten die Tiere in einen Erregungszustand, welcher sich in gelegentlichen kurzen Zweikämpfen äussert. Man wählt Mäusepaare, welche während einer 2minütigen Versuchsperiode mindestens fünfmal gekämpft haben, verabreicht ihnen die Versuchssubstanz auf oralem Wege und wiederholt den Versuch nach 1 h. Hierbei verwendet man logarithmisch ansteigende Dosen bis zu maximal 100 mg/kg. Man definiert als 100%ige hemmende Dosis diejenige Dosis, welche bei 3 von 3 Mäusepaaren einen Kampf verhindert.

*Test an der geneigten Ebene:*

Man verabreicht jeweils 6 Mäusen die Prüfsubstanz (maximale Dosis ist 500 mg/kg) und beobachtet sodann während 4 h das Verhalten der Mäuse an der geneigten Ebene auf paralytische Erscheinungen, die ein Abgleiten von der Ebene verursachen. Wird eine Wirkung beobachtet, so verwendet man weitere Dosen, bis man mindestens zwei Dosen hat, bei welchen einige, jedoch nicht alle Tiere, von der geneigten Ebene abgleiten. Dosen, bei welchen die Tiere auf Grund von toxischen Erscheinungen oder Erregungszuständen abgleiten, werden für die Verrechnung des $PD_{50}$-Wertes nicht berücksichtigt.

*Versuch an der wachen Katze:*

Man behandelt Katzen auf oralem Wege und beobachtet, ob Symptome auftreten (gewöhnlich Ataxis). Zunächst verwendet man eine Katze und eine Dosis von 50 mg/kg. Wenn sich eine Wirkung zeigt, so variiert man die Dosen und benützt bis zu 3 Kapseln pro Dosis. Die Resultate zeigen die minimale wirksame Dosis.

Die nachfolgenden Beispiele sollen die Erfindung illustrieren, jedoch nicht einschränken. Alle Temperaturen sind, sofern nichts anderes erwähnt, in Celsiusgraden angegeben.

*Beispiel 1:*

Ein Fisher-Porter-Druckgefäss, enthaltend 12,5 g (33,8 mmol) 5-Brom-8-chlor-1-phenyl-3H-2-benzazepinhydrochlorid, 175 mg (0,02 mmol) Dibrombis(triphenylphosphin)palladium(II) und 250 mg (1,3 mmol) Kupfer(I)jodid, wird mit Argon ausgespült. Anschliessend führt man 17 ml Tri-n-butylamin und 6,2 ml n-Butanol ein und erhitzt die Mischung unter einem Kohlenstoffmonoxiddruck von 40 psi (2,75 · $10^5$ Pascal) während 18 h auf 110°. Man kühlt ab, verdünnt mit Äther und wäscht mit Wasser. Die Ätherlösung wird über wasserfreiem Natriumsulfat getrocknet und unter vermindertem Druck eingedampft, wobei man 13,0 g eines roten Öls erhält. Man reinigt 4,0 g dieses Öles durch Säulenchromatographie an 40 g Kieselgel unter Eluieren mit Methylenchlorid und erhält 8-Chlor-1-phenyl-5H-2-benzazepin-5-carbonsäurebutylester als gelbes Öl und, nach weiterem Eluieren mit Methylenchlorid, 8-Chlor-1-phenyl-3H-2-benzazepin-5-carbonsäurebutylester als gelbes Öl.

*Beispiel 2:*

Ein Fisher-Porter-Druckgefäss, enthaltend 12,8 g (33 mmol) 5-Brom-8-Chlor-1-(2-fluorphenyl)-3H-2-benzazepinhydrochlorid, 175 mg (0,2 mmol) Dibrombis(triphenylphosphin)palladium(II) und 250 mg (1,3 mmol) Kupfer(I)jodid, wird mit Argon ausgespült. Anschliessend führt man 17 ml Tri-n-butylamin und 6,2 ml n-Butanol ein und erhitzt die Mischung unter einem Kohlenstoffmonoxiddruck von 40 psi (2,75 · $10^5$ Pascal) während 18 h auf 110°. Man kühlt ab, verdünnt mit Äther und wäscht mit Wasser. Die Ätherlösung wird über wasserfreiem Natriumsulfat getrocknet und unter vermindertem Druck eingedampft, wobei man eine Mischung von 8-Chlor-1-(2-fluorphenyl)-5H-2-benzazepin-5-carbonsäurebutylester und 8-Chlor-1-(2-fluorphenyl)-3H-2-benzazepin-5-carbonsäurebutylester als rotes Öl erhält. Dieses Öl wird ohne weitere Reinigung in die nächste Stufe eingesetzt.

*Beispiel 3:*

Ein Fisher-Porter-Druckgefäss, enthaltend 3,5 g (9,5 mmol) 5-Brom-8-chlor-1-(2-chlorphenyl)-3H-2-benzazepin und 70 mg (0,09 mmol) Dibrombis(triphenylphosphin)palladium(II), wird mit Argon ausgespült. Anschliessend führt man 1,8 ml n-Butanol und 2,6 ml Tri-n-butylamin ein und erhitzt die Mischung unter einem Kohlenmonoxiddruck von 40 psi (2,75 · $10^5$ Pascal) während 24 h auf 100°. Man kühlt ab, verdünnt mit Äther und wäscht mit Wasser. Die Ätherlösung wird über wasserfreiem Natriumsulfat getrocknet und unter vermindertem Druck eingedampft, wobei man eine Mischung von 8-Chlor-1-(2-chlorphenyl)-5H-2-benzazepin-5-carbonsäurebutylester und 8-Chlor-1-(2-chlorphenyl)-3H-2-benzazepin-5-carbonsäurebutylester als rotes Öl erhält. Dieses Öl wird ohne weitere Reinigung in die nächste Stufe eingesetzt.

*Beispiel 4:*

Ein Fisher-Porter-Druckgefäss, enthaltend 2,0 g (5,1 mmol) 5-Brom-8-chlor-1-(2-fluorphenyl)-3H-2-benzazepinhydrochlorid, 50 mg (0,06 mmol) Dibrombis(triphenylphosphin)palladium(II) und 60 mg (0,3 mmol) Kupfer(I)jodid, wird mit Argon ausgespült. Anschliessend führt man 2,6 ml Tri-n-butylamin und 2,0 ml Methanol ein und erhitzt die Mischung unter einem Kohlenmonoxiddruck von 40 psi (2,75 · $10^5$ Pascal) während 18 h auf 100°. Man kühlt ab, verdünnt mit Äther und wäscht mit Wasser. Die Ätherlösung wird über wasserfreiem Natriumsulfat getrocknet und unter vermindertem Druck eingedampft. Man reinigt den Rückstand durch Säulenchromatographie an 30 g Kieselgel unter Eluieren mit Methylenchlorid und erhält als erstes Produkt 8-Chlor-2-(2-fluorphenyl)-3H-2-benzazepin-5-carbonsäuremethylester als weissliche Nadeln vom Schmelzpunkt 106-107°.

Durch weiteres Eluieren mit 20% Methylenchlorid (v/v) erhält man 8-Chlor-1-(2-fluorphenyl)-5H-2-benzazepin-5-carbonsäuremethylester als hellgelbe Nadeln vom Schmelzpunkt 125-126°.

*Beispiel 5:*

In einer Portion gibt man 11,5 ml (49 mmol) einer 4,34M-Lösung von Natriummethoxid in Methanol zu einer Lösung von 11,0 g (31 mmol) einer Mischung von 8-Chlor-1-phenyl-5H-2-benzazepin-5-carbonsäurebutylester und 8-Chlor-1-phenyl-3H-2-benzazepin-5-carbonsäurebutylester und 5,7 g (60 mmol) Acetamidinhydrochlorid in 200 ml Äthanol, erhitzt die Mischung während 18 h unter Rückfluss zum Sieden, kühlt ab und giesst auf 800 ml Wasser. Der erhaltene Niederschlag wird abfiltriert, mit Äther gewaschen und liefert einen gelben Festkörper. Durch Umkristallisieren aus Tetrahydrofuran erhält man 9-Chlor-4a,11b-dihydro-3-methyl-7-phenyl-5H-pyrimido-[4,5]-[2]-benzazepin-1(2H)-on als weissliche Prismen vom Schmelzpunkt 155-160°.

*Beispiel 6:*

In einer Portion gibt man 14,0 ml einer 4,34M-Lösung von Natriummethoxid in Methanol zu einer Lösung von 14,0 g (37,7 mmol) einer Mischung von 8-Chlor-1-(2-fluorphenyl)-5H-2-benzazepin-5-carbonsäurebutylester und 8-Chlor-1-(2-fluorphenyl)-3H-2-benzazepin-5-carbonsäurebutylester und 7,0 g (74 mmol) Acetamidinhydrochlorid in 250 ml Äthanol, erhitzt die Mischung während 17 h unter Rückfluss zum Sieden, kühlt ab und giesst auf 1 l Wasser. Der erhaltene Niederschlag wird abfiltriert und mit Äther gewaschen. Man erhält 9-Chlor-7-(2-fluorphenyl)-4a,11b-dihydro-3-methyl-5H-pyrimido-[4,5]-[2]-benzazepin-1(2H)-on als weisslichen Festkörper vom Schmelzpunkt 150-165°.

Anstelle einer Mischung der Butylester kann man auch die gemäss Beispiel 4 erhaltenen Methylester oder eine Mischung davon einsetzen.

*Beispiel 7:*

Man gibt 9,5 ml (41 mmol) einer 4,3M-Lösung von Natriummethoxid in Methanol tropfenweise zu einer Lösung von 8,0 g (22,6 mmol) einer Mischung von 8-Chlor-1-phenyl-5H-2-benzazepin-5-carbonsäurebutylester und 8-Chlor-1-phenyl-3H-2-benzazepin-5-carbonsäurebutylester und 6,5 g (62,4 mmol) Formamidinacetat in 70 ml Äthanol, rührt nach beendeter Zugabe noch während 30 min bei Raumtemperatur, verdünnt mit Wasser und extrahiert die Mischung mit Methylenchlorid. Die Methylenchloridlösung wird mit Wasser gewaschen, über wasserfreiem Natriumsulfat getrocknet und unter vermindertem Druck eingedampft. Durch Kristallisieren des Rückstandes aus einer Mischung von Äther und Petroläther erhält man einen gelben Festkörper, den man aus einer Mischung von Äther und Methylenchlorid umkristallisiert. Man erhält 9-Chlor-4a,11b-dihydro-7-phenyl-5-pyrimido-[4,5-d]-[2]-benzazepin-1(2H)-on (kristallisiert mit ⅓ mol Wasser) als hellgelbe Prismen vom Schmelzpunkt 150-155°.

Die Mutterlaugen werden unter vermindertem Druck eingedampft. Den erhaltenen Rückstand reinigt man mittels Filtration durch Kieselgel unter Eluieren mit Methylenchlorid und erhält 8-Chlor-1-phenyl-5H-2-benzazepin-5-carbonsäurebutylester (eines der beiden Ausgangsstoffe), das im Dünnschichtchromatogramm den gleichen Rf-Wert aufweist wie eine authentische Probe.

*Beispiel 8:*

Eine Mischung von 20,0 g (230 mmol) aktiviertem Mangandioxid und 5,3 g (14,8 mmol) 9-Chlor-4a,11b-dihydro-3-methyl-7-phenyl-5H-pyrimido-[4,5-d]-[2]-benzazepin-1(2H)-on in 500 ml Tetrahydrofuran wird während 2 h unter Rückfluss zum Sieden erhitzt. Man filtriert durch Kieselgur, um das Mangandioxid zu entfernen, und dampft das Filtrat unter vermindertem Druck ein. Man erhält 9-Chlor-3-methyl-7-phenyl-5H-pyrimido-[4,5-d]-[2]-benzazepin-1(2H)-on als hellgelben Festkörper vom Schmelzpunkt 245-248°. Durch Umkristallisieren aus einer Mischung von Methylenchlorid und Äther erhält man farblose Kristalle vom Schmelzpunkt 248-249°.

*Beispiel 9:*

9-Chlor-7-(2-fluorphenyl-3-methyl-5H-pyrimido-[4,5-d]-[2]-benzazepin-1(2H)-on wird auf die gleiche Weise hergestellt wie 9-Chlor-3-methyl-7-phenyl-5H-pyrimido-[4,5-d]-[2]-benzazepin-1(2H)-on. Man erhält hellgelbe Prismen vom Schmelzpunkt 262-264°.

*Beispiel 10:*

In einer Portion gibt man 6,0 ml (24 mmol) einer 4,0M-Lösung von Natriummethoxid in Methanol zu 4,35 g (11,2 mmol) einer Mischung von 8-Chlor-1-(2-chlorphenyl)-5H-2-benzazepin-5-carbonsäurebutylester und 8-Chlor-1-(2-chlorphenyl)-3H-2-benzazepin-5-carbonsäurebutylester und 2,5 g (26 mmol) Acetamidinhydrochlorid in 50 ml Äthanol, erhitzt die Mischung während 6 h unter Rückfluss zum Sieden und verdünnt mit Wasser. Das ausgefallene Material wird abfiltriert und mit Äther gewaschen, wobei man einen amorphen bräunlichen Festkörper erhält.

Eine Mischung dieses bräunlichen Festkörpers mit 6,0 g aktiviertem Mangandioxid in 100 ml Tetrahydrofuran wird während 4 h unter Rückfluss zum Sieden erhitzt. Man filtriert anschliessend durch Kieselgur, um das Mangandioxid zu entfernen, und dampft das Filtrat anschliessend unter vermindertem Druck ein. Umkristallisieren des erhaltenen crèmefarbenen Festkörpers aus Essigester liefert 9-Chlor-7-(2-chlorphenyl)-3-methyl-5H-pyrimido-[4,5-d]-[2]-benzazepin-1(2H)-on als farblose Kristalle vom Schmelzpunkt 277-279°.

*Beispiel 11:*

Eine Mischung von 2,2 g (6,8 mmol) 9-Chlor-4a,11b-dihydro-7-phenyl-5H-pyrimido-[4,5-d]-

[2]-benzazepin-1(2H)-on und 10 g (110 mmol) aktiviertem Mangandioxid in 100 ml Tetrahydrofuran wird während 18 h unter Rückfluss zum Sieden erhitzt. Nach dem Abkühlen filtriert man durch Kieselgur, um das Mangandioxid zu entfernen, und dampft das Filtrat unter vermindertem Druck ein. Man reinigt den Rückstand durch Säulenchromatographie an 10 g Kieselgel unter Eluieren mit 20% Äther in Methylenchlorid und mit 50% Essigester in Methylenchlorid und erhält 9-Chlor-7-phenyl-5H-pyrimido-[4,5-d]-[2]-benzazepin-1(2H)-on als hellgelbe Prismen vom Schmelzpunkt 226-229°.

*Beispiel 12:*

In einer Portion gibt man 5,0 ml (22 mmol) einer 4,34M-Lösung von Natriummethoxid in Methanol zu einer Mischung von 5,0 g (13,5 mmol) einer Mischung von 8-Chlor-1-(2-fluorphenyl)-5H-2-benzazepin-5-carbonsäurebutylester und 8-Chlor-1-(2-fluorphenyl)-3H-2-benzazepin-5-carbonsäurebutylester und 5,0 g (27,7 mmol) Guanidincarbonat in 100 ml Äthanol, erhitzt die Mischung während 1 h unter Rückfluss zum Sieden und verdünnt mit Wasser. Das ausgefallene Material wird abfiltriert und mit Äther gewaschen; man erhält einen braunen Festkörper.

Eine Mischung dieses braunen Festkörpers und 5,0 g aktiviertem Mangandioxid in 175 ml Dimethylformamid wird während 2 h auf 100° erhitzt. Man filtriert durch Kieselgur, um das Mangandioxid zu entfernen, und verteilt das Filtrat zwischen Methylenchlorid und Wasser. Die Methylenchloridlösung wird über wasserfreiem Natriumsulfat getrocknet und unter vermindertem Druck zu einem bräunlichen Festkörper eingedampft. Durch Umkristallisieren aus einer Mischung von Methanol und Tetrahydrofuran erhält man 3-Amino-9-chlor-7-(2-fluorphenyl)-5H-pyrimido-[4,5-d]-[2]-benzazepin-1(2H)-on als weissliche Prismen vom Schmelzpunkt 331-332°.

*Beispiel 13:*

Eine Lösung von 3,5 g (10,4 mmol) 9-Chlor-3-methyl-7-phenyl-5H-pyrimido-[4,5-d]-[2]-benzazepin-1(2H)-on und 22 ml Phosphoroxychlorid in 100 ml Methylenchlorid wird während 18 h bei Raumtemperatur gerührt. Nach Eindampfen der Reaktionsmischung unter vermindertem Druck wird der erhaltene feste Rückstand zwischen eiskalter gesättigter Natriumcarbonatlösung und Methylenchlorid verteilt. Die Methylenchloridlösung wird über wasserfreiem Natriumsulfat getrocknet und unter vermindertem Druck zu einem gelben Rückstand eingedampft. Reinigung durch Säulenchromatographie an 40 g Kieselgel unter Eluieren mit Methylenchlorid/Äther (10:1) liefert 1,9-Dichlor-3-methyl-7-phenyl-5H-pyrimido-[4,5-d]-[2]-benzazepin als hellgelben Festkörper vom Schmelzpunkt 190-192°. Eine Probe dieses Materials wird aus einer Mischung von Äther und Petroläther umkristallisiert und liefert farblose Prismen vom Schmelzpunkt 191-193°.

*Beispiel 14:*

Eine Mischung von 3,3 g (9,3 mmol) 9-Chlor-7-(2-fluorphenyl)-3-methyl-5H-pyrimido-[4,5-d]-[2]-benzazepin-1(2H)-on und 20 ml Phosphoroxychlorid in 90 ml Methylenchlorid wird während 18 h bei Raumtemperatur gerührt. Nach Eindampfen der Reaktionsmischung unter vermindertem Druck wird der Rückstand zwischen wässerigem Natriumcarbonat und Methylenchlorid verteilt. Die Methylenchloridlösung wird über wasserfreiem Natriumsulfat getrocknet und unter vermindertem Druck eingedampft. Den erhaltenen Rückstand reinigt man durch Säulenchromatographie an 40 g Kieselgel unter Eluieren mit 10% Äther in Methylenchlorid und erhält 1,9-Dichlor-7-(2-fluorphenyl)-3-methyl-5H-pyrimido-[4,5-d]-[2]-benzazepin als gelbes Öl.

Das entsprechende Hydrochlorid erhält man, indem man ein Äquivalent einer 1,4M-Lösung von Chlorwasserstoff in Methanol zu einer Lösung von 1,9-Dichlor-7-(2-fluorphenyl)-3-methyl-5H-pyrimido-[4,5-d]-[2]-benzazepin in Äther gibt und das ausgefallene Material abfiltriert. Durch Umkristallisieren von 1,9-Dichlor-7-(2-fluorphenyl)-3-methyl-5H-pyrimido-[4,5-d]-[2]-benzazepinhydrochlorid aus einer Mischung von Methylenchlorid und Äther erhält man hellgelbe Prismen vom Schmelzpunkt 178-182°.

*Beispiel 15:*

Eine Lösung von 1,0 g (2,8 mmol) 1,9-Dichlor-3-methyl-7-phenyl-5H-pyrimido-[4,5-d]-[2]-benzazepin und 1,0 ml einer 4,2M-Lösung von Natriummethoxid in Methanol in 20 ml einer 1:1-Mischung von Methanol und Tetrahydrofuran wird während 1 h bei Raumtemperatur gerührt. Man entfernt das Lösungsmittel unter vermindertem Druck und behandelt den Rückstand mit Wasser. Durch Umkristallisieren des erhaltenen weisslichen Festkörpers aus einer Mischung von Äther und Petroläther erhält man 9-Chlor-1-methoxy-3-methyl-7-phenyl-5H-pyrimido-[4,5-d]-[2]-benzazepin als farblose Prismen vom Schmelzpunkt 185-187°.

*Beispiel 16:*

9-Chlor-7-(2-fluorphenyl)-1-methoxy-3-methyl-5H-pyrimido-[4,5-d]-[2]-benzazepin wird auf die gleiche Weise hergestellt wie 9-Chlor-1-methoxy-3-methyl-7-phenyl-5H-pyrimido-[4,5-d]-[2]-benzazepin. Man erhält hellgelbe Prismen vom Schmelzpunkt 188-189°.

*Beispiel 17:*

In einer Portion gibt man 1,0 g (2,8 mmol) 1,9-Dichlor-3-methyl-7-phenyl-5H-pyrimido-[4,5-d]-[2]-benzazepin zu einer Lösung von 150 mg (3,1 mmol) Natriumhydrid (50%ig. Dispersion in Mineralöl) und einem Überschuss an Methylmercaptan (3,1 mmol) in 25 ml Dimethylformamid. Man rührt die Mischung während 30 min bei Raumtemperatur, verdünnt anschliessend mit Wasser und filtriert den erhaltenen Niederschlag ab. Durch Umkristallisieren des gelben Festkörpers aus einer Mischung von Äther und Methylenchlorid erhält man 9-Chlor-3-methyl-1-(methylthio)-7-phenyl-5H-pyrimido-[4,5-d]-[2]-benzazepin als hellgelbe Prismen vom Schmelzpunkt 181-183°.

*Beispiel 18:*

Eine Lösung von 1,0 g (2,8 mmol) 1,9-Dichlor-3-methyl-7-phenyl-5H-pyrimido-[4,5-d]-[2]-benzazepin und 1 ml Dimethylaminopropylamin in 25 ml Dimethylformamid wird während 16 h auf 65° erhitzt. Man giesst die Mischung anschliessend auf Eiswasser und filtriert den entstandenen Niederschlag ab. Durch Umkristallisieren des bräunlichen Festkörpers aus einer Mischung von Äther und Petroläther erhält man N-(9-Chlor-3-methyl-7-phenyl-5-pyrimido-[4,5-d]-[2]-benzazepin-1-yl)-N',N'-dimethyl-1,3-propandiamin (kristallisiert mit ⅓ mol Wasser) als farblose Nadeln vom Schmelzpunkt 124-127°.

Das Hydrochlorid wird hergestellt, indem man ein Äquivalent einer 1,4M-Lösung von Chlorwasserstoff in Methanol zu einer Lösung von N-(9-Chlor-3-methyl-7-phenyl-5H-pyrimido-[4,5-d]-[2]-benzazepin-1-yl)-N',N'-dimethyl-1,3-propandiamin in Methanol gibt und das entstandene Salz durch Zugabe von Äther ausfällt. Durch Umkristallisieren aus einer Mischung von Methanol und Äther erhält man N-(9-Chlor-3-methyl-7-phenyl-5H-pyrimido-[4,5-d]-[2]-benzazepin-1-yl)-N',N'-dimethyl-1,3-propandiaminhydrochlorid als weissliche Prismen vom Schmelzpunkt 190-194°.

*Beispiel 19:*

Man gibt 13,2 ml (18,5 mmol) einer 1,4M-Lösung von Methyllithium in Äther tropfenweise zu einer auf 0° gekühlten Suspension von 1,7 g (8,9 mmol) Kupfer(I)jodid in 50 ml Äther. Die erhaltene Ätherlösung von Lithiumdimethylcuprat wird auf −25° abgekühlt und tropfenweise mit einer Lösung von 0,8 g (2,2 mmol) 1,9-Dichlor-3-methyl-7-phenyl-5H-pyrimido-[4,5-d]-[2]-benzazepin in 30 ml Äther versetzt. Man rührt die Mischung während 1 h bei −25°, erwärmt auf Raumtemperatur, verdünnt mit Wasser und sättigt mit Schwefelwasserstoff. Man filtriert durch Kieselgur, um das ausgefallene Material zu entfernen, und dampft das Filtrat unter vermindertem Druck ein. Den Rückstand reinigt man durch Säulenchromatographie an 10 g Kieselgel unter Eluieren mit 20% Äther in Methylenchlorid und erhält ein Öl, das man aus einer Mischung von Äther und Petroläther kristallisiert. Man erhält 9-Chlor-1,3-dimethyl-7-phenyl-5H-pyrimido-[4,5-d]-[2]-benzazepin (kristallisiert mit ¼ mol Äther) als weissliche Nadeln vom Schmelzpunkt 121-124°.

*Beispiel 20:*

Eine Mischung von 2,0 g (5 mmol) 1-2-Benzoyl-(4-chlorphenyl)-3-phthalimidopropin und 0,1 g 10%ig. Palladium auf Bariumsulfat (vorhydriert) in 50 ml Tetrahydrofuran wird so lange bei Atmosphärendruck und Raumtemperatur hydriert, bis 85 ml Wasserstoff aufgenommen worden sind. Man filtriert vom Katalysator ab und dampft das Filtrat unter vermindertem Druck zur Trockene ein. Durch Kristallisieren des Rückstandes aus Äther erhält man einen weissen Festkörper vom Schmelzpunkt 70-72°. Umkristallisieren aus Äther liefert 1-2-Benzoyl-(4-chlorphenyl)-3-phthalimidopropen als farblose Prismen vom Schmelzpunkt 70-72°.

*Beispiel 21:*

Eine Mischung von 6 g (15 mmol) 1-2-Benzoyl-(4-chlorphenyl)-3-phthalimidopropen, 0,9 g (18 mmol) 85%ig. Hydrazinhydrat und 70 ml 95%ig. Äthanol wird während 2,5 h unter Rückfluss zum Sieden erhitzt. Man filtriert, um unlösliche Anteile zu entfernen, stellt das Filtrat mit eiskalter verdünnter Salzsäure sauer und extrahiert mit Äther. Die wässerige Phase wird abgetrennt, mit verdünnter Natronlauge alkalisch gestellt und mit Methylenchlorid extrahiert. Man trocknet die Methylenchloridlösung über wasserfreiem Natriumsulfat, stellt mit methanolischem Chlorwasserstoff sauer, verdünnt mit Isopropanol und engt unter vermindertem Druck zu einem kleinen Volumen ein. Das ausgefallene rohe 8-Chlor-1-phenyl-3H-2-benzazepinhydrochlorid wird abfiltriert. Man erhält bräunliche Prismen vom Schmelzpunkt 223-225° (Zersetzung).

*Beispiel 22:*

Eine Lösung von 2,8 g (8,3 mmol) 9-Chlor-3-methyl-7-phenyl-5H-pyrimido-[4,5-d]-[2]-benzazepin-1(2H)-on und 2,5 g (11,5 mmol) 85%ig. m-Chlorperbenzoesäure in 110 ml Methylenchlorid wird während 2,5 h bei Raumtemperatur gerührt. Man wäscht die Reaktionsmischung mit gesättigter wässeriger Natriumcarbonatlösung, trocknet über wasserfreiem Natriumsulfat und dampft im Vakuum zu einem gelben Festkörper ein. Durch Umkristallisieren aus Methanol erhält man 9-Chlor-3-methyl-7-phenyl-5H-pyrimido-[4,5-d]-[2]-benzazepin-1(2H)-on-6-oxid als hellgelbe Prismen vom Schmelzpunkt 219-221°.

*Beispiel 23:*

Eine Mischung von 0,5 g (1,4 mmol) 9-Chlor-3-methyl-7-phenyl-5H-pyrimido-[4,5-d]-[2]-benzazepin-1(2H)-on-6-oxid und 10 ml Essigsäureanhydrid wird auf einem Dampfbad während 3 h erwärmt. Man dampft unter vermindertem Druck ein und kristallisiert den erhaltenen kristallinen Festkörper aus Methylenchlorid um. Man erhält 5-(Acetyloxyloxy)-9-chlor-3-methyl-7-phenyl-5H-pyrimido-[4,5-d]-[2]-benzazepin-1(2H)-on als crèmefarbene Kristalle vom Schmelzpunkt >320°.

*Beispiel 24:*

Eine Mischung von 2,1 g (5,3 mmol) 5-(Acetyloxy)-9-chlor-3-methyl-7-phenyl-5H-pyrimido-[4,5-d]-[2]-benzazepin-1(2H)-on und 50 ml 3N-Natronlauge wird während 5 min auf einem Dampfbad erhitzt. Man verdünnt die Reaktionsmischung mit 200 ml Eiswasser und 50 ml Äther,

neutralisiert die Natronlauge mit Essigsäure und rührt die erhaltene Mischung während 30 min bei Raumtemperatur. Das ausgefallene Material wird abfiltriert. Durch Umkristallisieren des weisslichen Festkörpers aus Tetrahydrofuran erhält man 9-Chlor-5-hydroxy-3-methyl-7-phenyl-5H-pyrimido-[4,5-d]-[2]-benzazepin-1(2H)-on als farblose Kristalle vom Schmelzpunkt 253-255° (Zersetzung).

*Beispiel 25:*

Eine Mischung von 4,6 g (15 mmol) 3-Amino-1-[2-(2-chlorbenzoyl)-4-chlorphenyl]-propin und 0,1 g Palladium auf Bariumsulfat (vorhydriert) in 30 ml Tetrahydrofuran wird so lange bei Raumtemperatur und Atmosphärendruck hydriert, bis 355 ml Wasserstoff aufgenommen worden sind. Man filtriert, um den Katalysator zu entfernen, und dampft das Filtrat unter vermindertem Druck ein. Der Rückstand wird aus Äther kristallisiert und liefert 8-Chlor-2-(2-chlorphenyl)-3H-2-benzazepin als crèmefarbenen Festkörper vom Schmelzpunkt 113-115°. Durch Umkristallisieren aus Äther erhält man crèmefarbene Prismen vom Schmelzpunkt 117-118°.

Das Methansulfonat von 8-Chlor-1-(2-chlorphenyl)-3H-2-benzazepin wird hergestellt, indem man eine Lösung dieses Stoffes in Methanol mit überschüssiger 1M-Lösung von Methansulfonsäure in Methanol versetzt und das entstandene Salz durch Zugabe von Äther ausfällt. Durch Umkristallisieren aus einer Mischung von Methanol und Äther erhält man 8-Chlor-1-(2-chlorphenyl)-3H-2-benzazepinmethansulfonat als farblose Plättchen vom Schmelzpunkt 201-202°.

*Beispiel 26:*

8-Chlor-1-(2-fluorphenyl)-3H-2-benzazepinhydrochlorid wird auf die gleiche Weise hergestellt wie 8-Chlor-2-(2-chlorphenyl)-3H-2-benzazepin. Man erhält weissliche Prismen vom Schmelzpunkt 210-212° (Zersetzung).

*Beispiel 27:*

Tropfenweise gibt man 200 ml (0,18 mol) einer 5%ig. Lösung von Brom in Methylenchlorid zu 26,5 g (0,1 mol) 8-Chlor-1-phenyl-3H-2-benzazepin in 300 ml Methylenchlorid. Man rührt die Mischung während 1 h bei Raumtemperatur, verdünnt mit einem Überschuss an gesättigter wässeriger Natriumcarbonatlösung und rührt während 15 min bei Raumtemperatur. Die Methylenchloridphase wird abgetrennt, über wasserfreiem Natriumsulfat getrocknet und mit einem Überschuss an methanolischem Chlorwasserstoff verdünnt. Man engt unter vermindertem Druck zu einem kleinen Volumen ein und fällt das Salz durch Zugabe von Äther aus. Man erhält 4,5-Dibrom-8-chlor-4,5-dihydro-1-phenyl-3H-2-benzazepin als farblose Kristalle vom Schmelzpunkt 164-165° (Zersetzung) Diese Verbindung besitzt bei 172-173° (Zersetzung) einen zweiten Schmelzpunkt.

Eine Lösung des obigen Salzes in Methanol wird mit verdünnter wässeriger Natronlauge neutralisiert. Die ausgefallenen Kristalle werden abfiltriert und aus Methanol umkristallisiert. Man erhält 4,5-Dibrom-8-chlor-4,5-dihydro-1-phenyl-3H-2-benzazepin als farblose Prismen vom Schmelzpunkt 113-115°.

*Beispiel 28:*

4,5-Dibrom-8-chlor-1-(2-fluorphenyl)-4,5-dihydro-3H-2-benzazepin wird auf die gleiche Weise hergestellt wie 4,5-Dibrom-8-chlor-4,5-dihydro-1-phenyl-3H-2-benzazepin. Man erhält 4,5-Dibrom-8-chlor-1-(2-fluorphenyl)-4,5-dihydro-3H-2-benzazepinhydrochlorid als farblosen Festkörper vom Schmelzpunkt 158-159° (Zersetzung) und die freie Base als farblose Prismen vom Schmelzpunkt 102-103°.

*Beispiel 29:*

4,5-Dibrom-8-chlor-1-(2-chlorphenyl)-4,5-dihydro-3H-2-benzazepin wird auf die gleiche Weise hergestellt wie 4,5-Dibrom-8-chlor-4,5-dihydro-1-phenyl-3H-2-benzazepin. Man erhält hellgelbe Prismen vom Schmelzpunkt 139° (Zersetzung).

*Beispiel 30:*

Eine Lösung von 24 g (53 mmol) 4,5-Dibrom-8-chlor-4,5-dihydro-1-phenyl-3H-2-benzazepinhydrochlorid in 1 l Methanol und 180 ml 10%ig. wässerige Natronlauge wird während 45 h bei Raumtemperatur gerührt. Nach Einengen der Mischung im Vakuum auf ein kleines Volumen wird der Rückstand mit Methylenchlorid extrahiert. Die Methylenchloridlösung wird über wasserfreiem Natriumsulfat getrocknet, mit einem Überschuss an methanolischem Chlorwasserstoff verdünnt und im Vakuum zur Trockene eingedampft. Durch Kristallisieren des Rückstandes aus einer Mischung von Isopropanol und Äther erhält man 5-Brom-8-chlor-1-phenyl-3H-2-benzazepinhydrochlorid als weisslichen Festkörper vom Schmelzpunkt 229-230°. Umkristallisieren aus Methylenchlorid liefert farblose Prismen vom Schmelzpunkt 230-235° (Zersetzung).

Die rohen Mutterlaugen werden mit verdünnter wässeriger Natronlauge basisch gestellt und mit Methylenchlorid extrahiert. Die Methylenchloridlösung wird über wasserfreiem Natriumsulfat getrocknet und im Vakuum eingedampft. Reinigung des Rückstandes durch Säulenchromatographie an Kieselgel unter Eluieren mit Methylenchlorid und anschliessend mit Äther liefert nach Eindampfen der Ätherfraktionen ein farbloses Öl. Man nimmt dieses Öl in einer Lösung von Methansulfonsäure in Methanol auf und fällt das entstandene Salz durch Zugabe von Äther aus. Durch Umkristallisieren aus einer Mischung von Methanol und Äther erhält man 8-Chlor-3-methoxy-1-phenyl-3H-2-benzazepinmethansulfonat als weissliche Prismen vom Schmelzpunkt 139-140°.

*Beispiel 31:*

Eine Mischung von 21 g (45 mmol) 4,5-Dibrom-8-chlor-1-(2-fluorphenyl)-4,5-dihydro-3H-2-benzazepinhydrochlorid, 40 ml Dioxan, 360 ml Methanol und 40 ml einer 10%ig. wässerigen Natronlauge wird während 5 h bei Raumtemperatur gerührt und anschliessend im Vakuum auf ein kleines Volumen eingeengt. Man verdünnt mit Wasser und extrahiert mit Methylenchlorid. Die Methylenchloridlösung wird über wasserfreiem Natriumsulfat getrocknet, mit Isopropanol und einem Überschuss an methanolischem Chlorwasserstoff verdünnt und unter vermindertem Druck zu einem kleinen Volumen eingeengt. Man erhält 5-Brom-8-chlor-1-(2-fluorphenyl)-3H-2-benzazepinhydrochlorid als farblosen Festkörper vom Schmelzpunkt 231-232°. Durch Umkristallisieren aus einer Mischung von Methylenchlorid und Äther erhält man farblose Kristalle vom Schmelzpunkt 233-234° (Zersetzung). Das Methansulfonatsalz des Nebenproduktes wird nicht isoliert.

*Beispiel 32:*

Eine Lösung von 60,0 g (0,134 mol) 4,5-Dibrom-8-chlor-1-(2-chlorphenyl-4,5-dihydro-3H-2-benzazepin und 75 ml 40%ig. wässeriger Natriumlauge in einer Mischung von 300 ml Dioxan und 900 ml Methanol wird während 4 h bei Raumtemperatur gerührt und anschliessend im Vakuum zu einem kleinen Volumen eingeengt. Man extrahiert den Rückstand mit Methylenchlorid, trocknet die Methylenchloridlösung über wasserfreiem Natriumsulfat, verdünnt mit einem Überschuss an methanolischem Chlorwasserstoff und Isopropanol und dampft im Vakuum zur Trockene ein. Den Rückstand kristallisiert man aus einer Mischung von Isopropanol in Äther, wobei man einen weissen Festkörper erhält, den man zwischen Methylenchlorid und wässerigem Natriumbicarbonat verteilt. Die Methylenchloridphase wird über wasserfreiem Natriumsulfat getrocknet und unter vermindertem Druck zu einem bernsteinfarbenen Öl eingedampft. Reinigung durch Säulenchromatographie an 250 g Kieselgel unter Eluieren mit Methylenchlorid liefert 5-Brom-8-chlor-1-(2-chlorphenyl)-3H-2-benzazepin als farblose Prismen vom Schmelzpunkt 125-127°.

Die rohen Mutterlaugen verteilt man zwischen Methylenchlorid und wässerigem Ammoniak, trocknet die Methylenchloridlösung über wasserfreiem Natriumsulfat und dampft unter vermindertem Druck ein. Durch Behandeln des Rückstandes mit einer Mischung von Äther und Petroläther erhält man 8-Chlor-1-(2-chlorphenyl)-3-methoxy-3H-2-benzazepin als bräunlichen Festkörper. Durch Umkristallisieren aus einer Mischung von Äther und Petroläther erhält man crèmefarbene Prismen vom Schmelzpunkt 83-85°.

*Beispiel 33:*

Eine Mischung aus 76 g (1,1 mol) Natriumnitrit und 450 ml Schwefelsäure wird auf dem Dampfbad auf ca. 80° erwärmt, bis vollständige Lösung eintritt. Nach Abkühlen auf 30° werden 232 g (1,0 mol) 2-Amino-5-chlorbenzophenon unter Rühren portionenweise so zugegeben, dass die Temperatur zwischen 30 und 40° bleibt. Nach 1 h wird die Mischung langsam zu 3 l Eiswasser gegeben und durch Kieselgur filtriert. Das Filtrat wird unter Rühren langsam mit einer Lösung von 200 g (1,83 mol) Natriumtetrafluoroborat in 800 ml Wasser versetzt. Der dabei entstehende Niederschlag wird abfiltriert und zweimal mit je 100 ml Wasser gewaschen.

Das feuchte 2-Benzoyl-4-chlorbenzoldiazoniumtetrafluoroborat wird in 3 l Wasser suspendiert und tropfenweise mit einer Lösung von 332 g (2 mol) Kaliumjodid in 1 l Wasser versetzt. Die Mischung wird während 4 h bei Raumtemperatur gerührt; anschliessend filtriert man das Rohprodukt ab und gibt es zu 1 l siedendem Äther. Nach Filtrieren und Trocknen über Natriumsulfat wird die ätherische Lösung auf 500 ml eingeengt. Durch Zugeben von 100 ml Petroläther erhält man 5-Chlor-2-jodbenzophenon. Eine Probe dieses Materials wird aus einer Mischung von Äther und Petroläther umkristallisiert und liefert hellgelbe Prismen vom Schmelzpunkt 80-82°.

*Beispiel 34:*

Die Herstellung von 5-Chlor-2'-fluor-2-jodbenzophenon erfolgt auf die gleiche Weise wie die Herstellung von 5-Chlor-2-jodbenzophenon und liefert hellgelbe Prismen vom Schmelzpunkt 78-81°.

*Beispiel 35:*

Die Herstellung von 2'-5-Dichlor-2-jodbenzophenon erfolgt auf die gleiche Weise wie die Herstellung von 5-Chlor-2-jodbenzophenon und liefert hellgelbe Prismen vom Schmelzpunkt 64-66°.

*Beispiel 36:*

Eine Mischung von 0,71 g (4,0 mmol) Palladiumchlorid, 2,1 g (8,0 mmol) Triphenylphosphin, 0,8 g (4,2 mmol) Kupfer(I)jodid, 68,8 g (0,21 mol) 5-Chlor-2-jodbenzophenon, 200 ml Diäthylamin und 400 ml Methylenchlorid wird bei Raumtemperatur unter Argon gerührt, bis alles in Lösung ist. In einer Portion gibt man 40,0 g (0,22 mol) N-Propargylphthalimid hinzu und rührt die entstandene Mischung während 20 h. Nach Entfernen der flüchtigen Anteile im Vakuum wird der Rückstand mit 200 ml Isopropanol verrieben. Durch Abfiltrieren des entstandenen Niederschlages erhält man rohes 1-(2-Benzoyl-4-chlorphenyl)-3-phthalimidopropin. Durch Umkristallisieren aus Aceton erhält man crèmefarbene Prismen vom Schmelzpunkt 148-150°.

*Beispiel 37:*

Die Herstellung von 1-[4-Chlor-2-(2-fluorbenzoyl)phenyl]-3-phthalimidopropin erfolgt auf die gleiche Weise wie die Herstellung von 1-(2-Benzoyl-4-chlorphenyl)-3-phthalimidopropin und liefert crèmefarbene Prismen vom Schmelzpunkt 158-161°.

*Beispiel 38:*

Die Herstellung von 1-[4-Chlor-2-(2-chlorbenzoyl)phenyl]-3-phthalimidopropin erfolgt auf die gleiche Weise wie die Herstellung von 1-(2-Benzoyl-4-chlorphenyl)-3-phthalimidopropin

und liefert crèmefarbene Prismen vom Schmelzpunkt 144-145°.

*Beispiel 39:*

*Methode A:*

Eine Mischung aus 50 g 1-[4-Chlor-2-(2-fluorbenzoyl)phenyl]-3-phthalimidopropin, 50 ml 40%ig. Methylaminlösung und 150 ml Dimethylformamid wird während 25 min bei Raumtemperatur gerührt. Tropfenweise gibt man 500 ml Wasser hinzu und filtriert den entstandenen Niederschlag ab. Der Niederschlag wird mit Methylenchlorid gelöst, über Natriumsulfat getrocknet und im Vakuum eingedampft, wobei ein schwach gelber Festkörper anfällt. Durch Umkristallisieren dieses Stoffes aus Äther erhält man 3-Amino-1-[4-chlor-2-(2-fluorbenzoyl)phenyl]propin als hellgelbe Prismen vom Schmelzpunkt 89-91°.

*Methode B:*

Eine Mischung von 400 g (0,96 mol) 1-[4-Chlor-2-(2-fluorbenzoyl)phenyl]-3-phthalimidopropin, 1,3 l Äthanol und 300 ml 40%ig. Methylaminlösung wird bei Raumtemperatur während 2 h gerührt. Tropfenweise gibt man 2,8 l Wasser hinzu und filtriert den entstandenen Niederschlag ab, wobei ein hellgelber Festkörper vom Schmelzpunkt 79-80° anfällt. Durch Umkristallisieren aus Äther erhält man 3-Amino-1-[4-chlor-2-(2-fluorbenzoyl)phenyl]propin als hellgelbe Prismen vom Schmelzpunkt 89-91°.

*Beispiel 40:*

Die Herstellung von 3-Amino-1-[4-chlor-2-(2-chlorbenzoyl)phenyl]propin erfolgt auf die gleiche Weise wie die Herstellung von 3-Amino-1-[4-chlor-2-(2-fluorbenzoyl)phenyl]propin und liefert hellgelbe Prismen vom Schmelzpunkt 81-82°.

*Beispiel 41*

Herstellung von Tabletten (direkte Verpressung)

| Bestandteile | mg/Tablette | mg/Tablette | mg/Tablette | mg/Tablette |
|---|---|---|---|---|
| 1. 9-Chlor-7-(2-fluorphenyl)-3-methyl-5H-pyrimido-[4,5-d]-[2]-benzazepin-1(2H)-on, oder 1,9-Dichlor-3-methyl-7-phenyl-5H-pyrimido-[4,5-d]-[2]-benzazepin | 1 | 5 | 10 | 25 |
| 2. Lactose | 221 | 217 | 212 | 181 |
| 3. Avicel | 45 | 45 | 45 | 55 |
| 4. Stärke für die direkte Verpressung | 30 | 30 | 30 | 35 |
| 5. Magnesiumstearat | 3 | 3 | 3 | 4 |
| Tablettengewicht | 300 | 300 | 300 | 300 |

Der Wirkstoff wird mit der gleichen Menge Lactose gut vermischt. Man gibt die Bestandteile 3 und 4 und den Rest von Bestandteil 2 hinzu und vermischt erneut. Man versetzt anschliessend mit dem Magnesiumstearat, vermischt während 3 min und verpresst dann zu Tabletten geeigneter Grösse.

*Beispiel 42*

Herstellung von Tabletten (Nassgranulierung)

| Bestandteile | mg/Tablette | mg/Tablette | mg/Tablette | mg/Tablette |
|---|---|---|---|---|
| 1. 9-Chlor-7-(2-fluorphenyl)-3-methyl-5H-pyrimido-[4,5-d]-[2]-benzazepin-1(2H)-on, oder 1,9-Dichlor-3-methyl-7-phenyl-5H-pyrimido-[4,5-d]-[2]-benzazepin | 1 | 5 | 10 | 25 |
| 2. Lactose | 202 | 232 | 261 | 280 |
| 3. Modifizierte Stärke | 25 | 35 | 45 | 55 |
| 4. Vorgelatinisierte Stärke | 20 | 25 | 30 | 35 |
| 5. Destilliertes Wasser q.s. | — | — | — | — |
| 6. Magnesiumstearat | 2 | 3 | 4 | 5 |
| Tablettengewicht | 250 | 300 | 350 | 400 |

Man vermischt die Bestandteile 1 bis 4 in einem geeigneten Mixer, granuliert mit genügend destilliertem Wasser, um eine geeignete Konsistanz zu erhalten, vermahlt und trocknet in einem geeigneten Ofen. Nach erneutem Mahlen vermischt man während 3 min mit dem Magnesiumstearat und verpresst anschliessend zu Tabletten geeigneter Grösse.

*Beispiel 43*

Herstellung von Kapseln

| Bestandteile | mg/Kapsel | mg/Kapsel | mg/Kapsel | mg/Kapsel |
|---|---|---|---|---|
| 1. 9-Chlor-7-(2-fluorphenyl)-3-methyl-5H-pyrimido-[4,5-d]-[2]-benzazepin-1(2H)-on, oder 1,9-Dichlor-3-methyl-7-phenyl-5H-pyrimido-[4,5-d]-[2]-benzazepin | 1 | 5 | 10 | 25 |
| 2. Lactose | 203 | 293,5 | 328 | 372,5 |
| 3. Stärke | 30 | 35 | 40 | 30 |
| 4. Talk | 15 | 15 | 20 | 20 |
| 5. Aerosol OT | 1 | 1,5 | 2 | 2,5 |
| Kapselfüllgewicht | 250 | 350 | 400 | 450 |

Man vermischt die Bestandteile 1, 2, 3 und 5 in einem geeigneten Mixer, vermahlt, versetzt mit dem Talk und vermischt gut. Die Masse wird anschliessend auf einer Kapselabfüllmaschine in Kapseln geeigneter Grösse abgefüllt.

**Patentansprüche**

1. Pyrimido-[4,5-d]-[2]-benzazepine der allgemeinen Formel

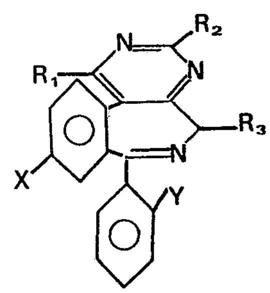

(I)

worin $R_1$ Wasserstoff, $C_1$- bis $C_7$-Alkyl, Chlor, Brom, $C_1$- bis $C_7$-Alkoxy, Hydroxy, $C_1$- bis $C_7$-Alkylthio oder $-NR_4R_5$, $R_4$ und $R_5$ Wasserstoff, $C_1$- bis $C_7$-Alkyl oder di-$C_1$- bis $C_7$-Alkylamino-$C_1$- bis $C_7$-Alkyl, $R_2$ Wasserstoff, $C_1$- bis $C_7$-Alkyl oder Amino, $R_3$ Wasserstoff, $C_1$- bis $C_6$-Alkanoyloxy, Trifluoracetoxy, Benzoyloxy oder Hydroxy, X Halogen mit einer Ordnungszahl, welche nicht grösser als 35 ist, und Y Wasserstoff oder Halogen mit einer Ordnungszahl, welche nicht grösser als 35 ist, bedeuten, und die 6-Oxide davon, wenn $R_3$ Wasserstoff bedeutet,
und pharmazeutisch annehmbare Salze davon.

2. Verbindungen gemäss dem Anspruch 1, dadurch gekennzeichnet, dass $R_3$ Wasserstoff und $R_2$ Wasserstoff oder $C_1$- bis $C_7$-Alkyl bedeutet, und pharmazeutisch annehmbare Säureadditionssalze davon.

3. 9-Chlor-3-methyl-7-phenyl-5H-pyrimido-[4,5-d]-[2]-benzazepin-1(2H)-on.

4. 9-Chlor-7-(2-fluorphenyl)-3-methyl-5H-pyrimido-[4,5-d]-[2]-benzazepin-1(2H)-on.

5. 1,9-Dichlor-3-methyl-7-phenyl-5H-pyrimido-[4,5-d]-[2]-benzazepin.

6. 9-Chlor-1,3-dimethyl-7-phenyl-5H-pyrimido-[4,5-d]-[2]-benzazepin.

7. 9-Chlor-7-(2-fluorphenyl)-3-methyl-1-methoxy-5H-pyrimido-[4,5-d]-[2]-benzazepin.

8. Verbindungen gemäss einem der Ansprüche 1 bis 7 als pharmazeutische Wirkstoffe.

9. Verbindungen gemäss einem der Ansprüche 1 bis 7 als anxiolytisch und sedativ wirksame Stoffe.

10. Verfahren zur Herstellung von Verbindungen gemäss einem der Ansprüche 1 bis 7, dadurch gekennzeichnet, dass man

a) eine Verbindung der allgemeinen Formel

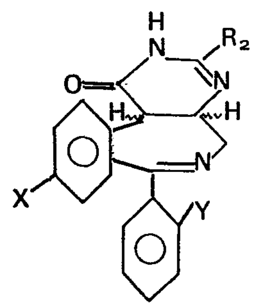

(VI)

worin $R_2$, X und Y die in Anspruch 1 angegebene Bedeutung besitzen,
dehydriert,
oder

b) eine Verbindung der in Anspruch 1 definierten Formel (I), worin $R_3$ Wasserstoff und $R_1$ Wasserstoff, $C_1$- bis $C_7$-Alkyl, Chlor, Brom, $C_1$- bis $C_7$-Alkoxy, Hydroxy oder $-NR_4R_5$ bedeuten, und $R_4$ und $R_5$ die in Anspruch 1 angegebene Bedeutung besitzen,
in 6-Stellung oxydiert, oder

c) ein 6-Oxid einer Verbindung der in Anspruch 1 definierten Formel (I), worin $R_3$ Wasserstoff bedeutet, mit einem einen $C_1$- bis $C_6$-Alkanoyl-, Trifluoracetyl- oder Benzoylrest liefernden Acylierungsmittel behandelt, oder

d) eine Verbindung der in Anspruch 1 definierten Formel (I), worin $R_3$ $C_1$- bis $C_6$-Alkanoyloxy, Trifluoracetoxy oder Benzoyloxy bedeutet, einer alkalischen Hydrolyse unterwirft, oder

e) eine Verbindung der in Anspruch 1 definierten Formel (I), worin $R_1$ Hydroxy und $R_3$ Wasserstoff bedeutet, chloriert oder bromiert, oder

f) eine Verbindung der in Anspruch 1 definierten Formel (I), worin $R_1$ Chlor oder Brom bedeutet, oder ein 6-Oxid davon, wenn $R_3$ Wasserstoff bedeutet, mit einem einen $C_1$- bis $C_7$-Alkyl-, $C_1$- bis $C_7$-Alkylmercapto- oder $C_1$- bis $C_7$-Alkoxyrest, oder einem die Gruppe $-NR_4R_5$ liefernden Mittel behandelt, oder

g) eine Verbindung der in Anspruch 1 definierten Formel (I), worin $R_1$ Chlor oder Brom bedeutet, oder ein 6-Oxid davon, wenn $R_3$ Wasserstoff bedeutet, reduziert, oder

h) eine Verbindung der in Anspruch 1 definierten Formel (I), worin $R_1$ $C_1$- bis $C_7$-Alkylmercapto und $R_3$ Wasserstoff bedeuten, entschwefelt, oder

i) eine Verbindung der in Anspruch 1 definierten Formel (I), worin $R_1$ $C_1$- bis $C_7$-Alkylmercapto und $R_3$ Wasserstoff bedeuten, mit einem Amin der Formel $HNR_4R_5$, worin $R_4$ und $R_5$ die in Anspruch 1 angegebene Bedeutung besitzen, behandelt, oder

j) eine Verbindung der in Anspruch 1 definierten Formel (I) oder ein 6-Oxid einer Verbindung der Formel (I), worin $R_3$ Wasserstoff bedeutet, in ein pharmazeutisch annehmbares Säureadditionssalz überführt.

11. Arzneimittel, enthaltend eine Verbindung gemäss einem der Ansprüche 1 bis 7.

12. Anxiolytika und Sedativa, enthaltend eine Verbindung gemäss einem der Ansprüche 1 bis 7.

## Claims

1. Pyrimido-[4,5-d]-[2]-benzazepines of the general formula

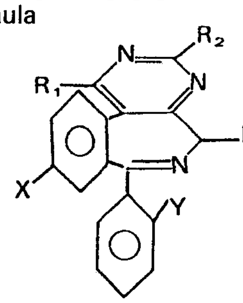

(I)

wherein $R_1$ signifies hydrogen, $C_1$-$C_7$-alkyl, chlorine, bromine, $C_1$-$C_7$-alkoxy, hydroxy, $C_1$-$C_7$-alkylthio or $-NR_4R_5$, $R_4$ and $R_5$ signify hydrogen, $C_1$-$C_7$-alkyl or di-$C_1$-$C_7$-alkylamino-$C_1$-$C_7$-alkyl, $R_2$ signifies hydrogen, $C_1$-$C_7$-alkyl or amino, $R_3$ signifies hydrogen, $C_1$-$C_6$-alkanoyloxy, trifluoroacetoxy, benzoyloxy or hydroxy, X signifies halogen with an atomic number which is not greater than 35, Y signifies hydrogen or halogen with an atomic number which is not greater than 35, and the 6-oxides thereof when $R_3$ signifies hydrogen,
and pharmaceutically acceptable salts thereof.

2. Compounds according to claim 1, characterized in that $R_3$ signifies hydrogen and $R_2$ signifies hydrogen or $C_1$-$C_7$-alkyl, and pharmaceutically acceptable acid addition salts thereof.

3. 9-Chloro-3-methyl-7-phenyl-5H-pyrimido-[4,5-d]-[2]-benzazepin-1(2H)-one.

4. 9-Chloro-7-(2-fluorophenyl)-3-methyl-5H-pyrimido-[4,5-d]-[2]-benzazepin-1(2H)-one.

5. 1,9-Dichloro-3-methyl-7-phenyl-5H-pyrimido-[4,5-d]-[2]-benzazepine.

6. 9-Chloro-1,3-dimethyl-7-phenyl-5H-pyrimido-[4,5-d]-[2]-benzazepine.

7. 9-Chloro-7-(2-fluorophenyl)-3-methyl-1-methoxy-5H-pyrimido-[4,5-d]-[2]-benzazepine.

8. Compounds according to any one of claims 1 to 7 as pharmaceutically active substances.

9. Compounds according to any one of claims 1 to 7 as anxiolytic and sedative active substances.

10. A process for the manufacture of compounds according to any one of claims 1 to 7, characterized by

(*a*) dehydrogenating a compound of the general formula

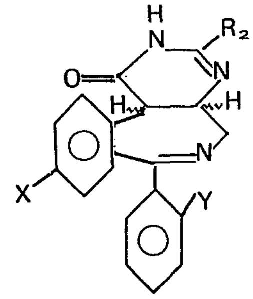

(VI)

wherein $R_2$, X and Y have the significance given in claim 1,
or

(*b*) oxidizing a compound of formula (I) defined in claim 1, wherein $R_3$ signifies hydrogen and $R_1$ signifies hydrogen, $C_1$-$C_7$-alkyl, chlorine, bromine, $C_1$-$C_7$-alkoxy, hydroxy or $-NR_4R_5$, and $R_4$ and $R_5$ have the significance given in claim 1, in the 6-position, or

(*c*) treating a 6-oxide of a compound of formula (I) defined in claim 1, wherein $R_3$ signifies hydrogen, with an acylating agent yielding a $C_1$-$C_6$-alkanoyl, trifluoroacetyl or benzoyl residue, or

(*d*) subjecting a compound of formula (I) defined in claim 1, wherein $R_3$ signifies $C_1$-$C_6$-alkanoyloxy, trifluoroacetoxy or benzoyloxy, to an alkaline hydrolysis, or

(*e*) chlorinating or brominating a compound of formula (I) defined in claim 1, wherein $R_1$ signifies hydroxy and $R_3$ signifies hydrogen, or

(*f*) treating a compound of formula (I) defined in claim 1, wherein $R_1$ signifies chlorine or bromine, or a 6-oxide thereof when $R_3$ signifies hydrogen, with an agent yielding a $C_1$-$C_7$-alkyl, $C_1$-$C_7$-akylmercapto or $C_1$-$C_7$-alkoxy residue or an agent yielding the group $-NR_4R_5$, or

(*g*) reducing a compound of formula (I) defined in claim 1, wherein $R_1$ signifies chlorine or bromine, or a 6-oxide thereof when $R_3$ signifies hydrogen, or

(*h*) desulphurizing a compound of formula (I) defined in claim 1, wherein $R_1$ signifies $C_1$-$C_7$-alkylmercapto and $R_3$ signifies hydrogen, or

(*i*) treating a compound of formula (I) defined in claim 1, wherein $R_1$ signifies $C_1$-$C_7$-alkylmercap-

to and $R_3$ signifies hydrogen, with an amine of the formula $HNR_4R_5$, wherein $R_4$ and $R_5$ have the significance given in claim 1, or

(*j*) converting a compound of formula (I) defined in claim 1 or a 6-oxide of a compound of formula (I), wherein $R_3$ signifies hydrogen, into a pharmaceutically acceptable acid addition salt.

11. Medicaments containing a compound according to any one of claims 1 to 7.

12. Anxiolytics and sedatives containing a compound according to any one of claims 1 to 7.

## Revendications

1. Pyrimido-[4,5-d]-[2]-benzazépines de formule générale:

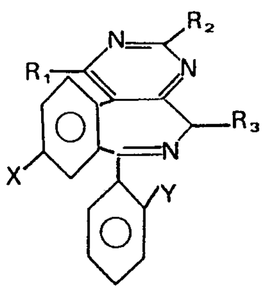

(I)

dans laquelle $R_1$ représente l'hydrogène, un groupe alkyle en $C_1$-$C_7$, le chlore, le brome, un groupe alcoxy en $C_1$-$C_7$, hydroxy, alkylthio en $C_1$-$C_7$ ou $-NR_4R_5$, $R_4$ et $R_5$ représentent l'hydrogène, des groupes alkyle en $C_1$-$C_7$ ou di(alkylamino en $C_1$-$C_7$)-alkyle en $C_1$-$C_7$, $R_2$ représente l'hydrogène, un groupe alkyle en $C_1$-$C_7$ ou un groupe amino, $R_3$ représente l'hydrogène, un groupe alcanoyloxy en $C_1$-$C_6$, trifluoracétoxy, benzoyloxy ou hydroxy, X représente un halogène de numéro atomique ne dépassant pas 35, et Y représente l'hydrogène ou un halogène de numéro atomique ne dépassant pas 35, et leurs 6-oxydes lorsque $R_3$ représente l'hydrogène,
et leurs sels acceptables pour l'usage pharmaceutique.

2. Composés selon la revendication 1, caractérisés en ce que $R_3$ représente l'hydrogène et $R_2$ l'hydrogène ou un groupe alkyle en $C_1$-$C_7$, et leurs sels formés par addition avec des acides acceptables pour l'usage pharmaceutique.

3. La 9-chloro-3-méthyl-7-phényl-5H-pyrimido-[4,5-d]-[2]-benzazépine-1(2H)-one.

4. La 9-chloro-7-(2-fluorophényl)-3-méthyl-5H-pyrimido-[4,5-d]-[2]-benzazépine-1(2H)-one.

5. La 1,9-dichloro-3-méthyl-7-phényl-5H-pyrimido-[4,5-d]-[2]-benzazépine.

6. La 9-chloro-1,3-diméthyl-7-phényl-5H-pyrimido-[4,5-d]-[2]-benzazépine.

7. La 9-chloro-7-(2-fluorophényl)-3-méthyl-1-méthoxy-5H-pyrimido-[4,5-d]-[2]-benzazépine.

8. Composés selon l'une des revendications 1 à 7, en tant que substances actives pharmaceutiques.

9. Composés selon l'une des revendications 1 à 7, en tant que substances actives anxiolytiques et sédatives.

10. Procédé de préparation des composés selon l'une des revendications 1 à 7, caractérisé en ce que:

a) on soumet un composé de formule générale:

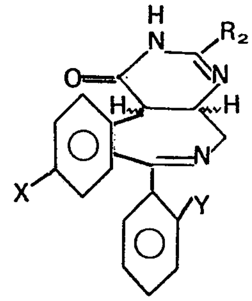

(VI)

dans laquelle $R_2$, X et Y ont les significations indiquées dans la revendication 1, à déshydrogénation, ou bien

b) on oxyde un composé de formule (I) définie dans la revendication 1, dans laquelle $R_3$ représente l'hydrogène et $R_1$ représente l'hydrogène, un groupe alkyle en $C_1$-$C_7$, le chlore, le brome, un groupe alcoxy en $C_1$-$C_7$, hydroxy ou $-NR_4R_5$, et $R_4$ et $R_5$ ont les significations indiquées dans la revendication 1, à oxydation en position 6, ou bien

c) on traite un 6-oxyde d'un composé de formule (I) définie dans la revendication 1, dans laquelle $R_3$ représente l'hydrogène, par un agent acylant fournissant un radical alcanoyle en $C_1$-$C_6$, trifluoracétyle ou benzoyle, ou bien

d) on soumet un composé de formule (I) définie dans la revendication 1, dans laquelle $R_3$ représente un groupe alcanoyloxy en $C_1$-$C_6$, trifluoracétoxy ou benzoyloxy, à une hydrolyse alcaline, ou bien

e) on soumet un composé de formule (I) définie dans la revendication 1, dans laquelle $R_1$ représente un groupe hydroxy et $R_3$ l'hydrogène, à chloration ou bromation, ou bien

f) on traite un composé de formule (I) définie dans la revendication 1, dans laquelle $R_1$ représente le chlore ou le brome, ou un 6-oxyde d'un tel composé lorsque $R_3$ représente l'hydrogène, par un agent fournissant un groupe alkyle en $C_1$-$C_7$, alkylmercapto en $C_1$-$C_7$ ou alcoxy en $C_1$-$C_7$ ou un agent fournissant le groupe $-NR_4R_5$, ou bien

g) on réduit un composé de formule (I) définie dans la revendication 1, dans laquelle $R_1$ représente le chlore ou le brome, ou un 6-oxyde d'un tel composé lorsque $R_3$ représente l'hydrogène, ou bien

h) on désulfure un composé de formule (I) définie dans la revendication 1, dans laquelle $R_1$ représente un groupe alkylmercapto en $C_1$-$C_7$ et $R_3$ l'hydrogène, ou bien

i) on traite un composé de formule (I) définie dans la revendication 1, dans laquelle $R_1$ représente un groupe alkylmercapto en $C_1$-$C_7$ et $R_3$ l'hydrogène, par une amine de formule $HNR_4R_5$ dans laquelle $R_4$ et $R_5$ ont les significations indiquées dans la revendication 1, ou bien

j) on convertit un composé de formule (I) définie dans la revendication 1, ou un 6-oxyde d'un

composé de formule (I), dans laquelle $R_3$ représente l'hydrogène, en un sel formé par addition avec un acide acceptable pour l'usage pharmaceutique.

11. Médicaments contenant un composé selon l'une des revendications 1 à 7.

12. Agents anxiolytiques et sédatifs contenant un composé selon l'une des revendications 1 à 7.